# EUROPEAN PATENT SPECIFICATION

(11) **EP 3 320 849 B1**
(45) Date of publication and mention of the grant of the patent: **30.10.2024**
(21) Application number: 17162673.2
(22) Date of filing: 23.03.2017
(51) Int. Cl.: A61B 8/08, A61B 8/00

(54) **ULTRASOUND IMAGING APPARATUS AND METHOD OF CONTROLLING THE SAME**
ULTRASCHALLBILDGEBUNGSVORRICHTUNG UND VERFAHREN ZUR STEUERUNG DAVON
APPAREIL D'IMAGERIE À ULTRASONS ET PROCÉDÉ DE COMMANDE CORRESPONDANT

(30) Priority: 15.11.2016 KR 20160151643
(43) Date of publication of application: 16.05.2018
(73) Proprietor: Samsung Medison Co., Ltd., Hongcheon-gun, Gangwon-do, 25108 (KR)
(72) Inventor: PARK, Sung Wook, Seoul (KR); LEE, Jin Yong, Seoul (KR); PARK, Jin Ki, Seoul (KR); YOON, Ji-Hyun, Seoul (KR); LEE, Sang-Eun, Seoul (KR); CHANG, Hyuk-Jae, Seoul (KR); CHUNG, Nam-Sik, Seoul (KR); CHO, In-Jeong, Seoul (KR)
(74) Representative: Hylarides, Paul Jacques

(56) References cited:
- EP-A1- 2 417 913
- EP-A1- 2 529 666
- US-A1- 2003 171 668
- US-A1- 2009 036 780
- US-A1- 2016 125 640

## Description

### BACKGROUND

### 1. Field

Embodiments of the present disclosure relate to an ultrasound imaging apparatus configured to generate an internal image of an object using ultrasonic waves.

### 2. Description of the Related Art

An ultrasound imaging apparatus is an apparatus that emits ultrasonic signals at a main body surface of an object toward a target part inside the main body and noninvasively obtains an image of a cross section of soft tissue or a blood flow using information of reflected ultrasonic signals (ultrasonic echo signals).

Compared with other image diagnosis apparatuses such as an X-ray diagnosis apparatus, an X-ray computerized tomography (CT) scanner, an magnetic resonance image (MRI), a nuclear medicine diagnosis apparatus, and the like, since the ultrasound imaging apparatus has a small size, is at a low price, is able to display in real time, and is free from radiation exposure with high safety, the ultrasound imaging apparatus is generally used for cardiac, abdominal, urinary, and obgyn diagnoses.

The ultrasound imaging apparatus includes an ultrasound probe configured to transmit ultrasonic signals to an object and receive ultrasonic echo signals reflected from the object to obtain an ultrasonic image of the object and a main body configured to generate an internal image of the object using the ultrasonic echo signals received at the ultrasound probe.

Various options are known to improve ultrasound imaging of the heart. EP 2529666 A1 teaches a display unit showing, aside from the ultrasonic image, calculated specific physical quantities. US 2016/0125640 A1 teaches a user interface configured to receive an input for setting a region of interest (ROI) in a first medical image and to set volume rendering properties differently for the ROI and the remaining region, with the display configured to display the resultant volume rendering. US 2003/0171668 A1 teaches a method to easily and accurately extract characterizing points from an ultrasound options. However, further improvement is desired.

### SUMMARY OF THE INVENTION

Therefore, it is an object of the present invention to provide an ultrasound imaging apparatus and method adapted to more efficiently and quickly recognize a cardiac structure- This object is achieved by an ultrasound imaging apparatus and method according to independent claims 1 and 9. Optional features are defined in the dependent claims.

### SUMMARY OF THE DISCLOSURE

Additional aspects of the present disclosure will be set forth in part in the description which follows and, in part, will be obvious from the description, or may be learned by practice of the present disclosure.

The ultrasonic image of the heart may be one of cardiac long axis cross sections of the heart.

The ROI image may then be at least one of cardiac short axis cross sections of the heart. The ultrasonic image of the heart may be one of cardiac short axis cross sections of the heart.

The ROI image may then be at least one of cardiac long axis cross sections of the heart. The ultrasonic image of the heart may be a stereoscopic image that three-dimensionally shows the heart.

The ROI image may then be at least one of a short axis cross section image and a long axis cross section image of the heart.

The controller may control the ultrasonic image of the heart and a guide line related to setting of the ROI in the ultrasonic image to be displayed together.
When the ROI of the ultrasonic image of the heart is selected through the input portion, the controller may control only an ultrasonic image of an area corresponding to the ROI selected from the ultrasonic image of the heart to be displayed.

The display portion may comprise a touch panel configured to receive a command for selecting the ROI of the ultrasonic image of the heart.

When the ROI of the ultrasonic image of the heart is selected through the display portion, the controller may control the ultrasonic image of the heart and the ROI image to be displayed on the display portion.

The controller may obtain and display a list of a plurality of such preset ROI images and controls at least one ROI image selected from the list of the plurality of displayed ROI images to be displayed.

The ultrasonic image of the heart and the at least one ROI image may be based on obtaining at different angles.

The ultrasonic image of the heart may be one of cardiac long axis cross sections of the heart.

The ROI image may then be at least one of cardiac short axis cross sections of the heart.

The ultrasonic image of the heart may be one of cardiac short axis cross sections of the heart.

The ROI image may then be at least one of cardiac long axis cross sections of the heart. The ultrasonic image of the heart may be a stereoscopic image that three-dimensionally shows the heart.

The ROI image may then be at least one of a short axis cross section image and a long axis cross section image of the heart.

The displaying of the ultrasonic image of the heart of the object may comprise, when the ROI of the ultrasonic image of the heart is selected, controlling only an ultrasonic image of an area corresponding to the ROI selected from the ultrasonic image of the heart to be displayed.

The method of controlling an ultrasound imaging apparatus, further comprising obtaining and displaying a list of a plurality of such preset ROI images and controlling at least one ROI image selected from the list of the plurality of displayed ROI images to be displayed.

### BRIEF DESCRIPTION OF THE DRAWINGS

These and/or other aspects of the present disclosure will become apparent and more readily appreciated from the following description of the embodiments, taken in conjunction with the accompanying drawings of which:
FIG. 1 is an external view of an ultrasound imaging apparatus in accordance with one embodiment
FIG. 2 is a control block diagram of the ultrasound imaging apparatus in accordance with one embodiment.
FIG. 3 is a control block diagram illustrating a configuration of a main body of the ultrasound imaging apparatus in accordance with one embodiment.
FIG. 4 is a view illustrating a long axis cross section of a heart.
FIG. 5 is a view illustrating a short axis cross section of a heart.
FIG. 6 is a view illustrating a long axis cross section and a short axis cross section of a heart that are illustrated on a display according to the disclosed embodiment.
FIG. 7 is a view illustrating a short axis cross section and a long axis cross section of a heart that are illustrated on a display according to the disclosed embodiment.
FIG. 8 is a view illustrating one long axis cross section and three short axis cross sections of a heart displayed on the display portion 550
FIG. 9 is a view illustrating one short axis cross section and three long axis cross sections of a heart displayed on one display portion.
FIG. 10 is a view illustrating that one stereoradiographic image and three short axis cross sections of a heart according to the disclosed embodiment are displayed on the display portion 550.
FIGS. 11A and 11B are views illustrating interfaces for inputting an ROI according to the disclosed embodiment.
FIGS. 12 and 13 are views illustrating ROIs according to the disclosed embodiment.
FIG. 14 is a flowchart according to the disclosed embodiment.

### DETAILED DESCRIPTION

Throughout the specification, like reference numerals refer to like elements. Throughout the specification, all of elements of embodiments are not described and well-known content in the art or repeated content among the embodiments will be omitted. The terms `portion, module, member, and block' used herein may be embodied as software or hardware. Depending on embodiments, a plurality of `portions, modules, members, or blocks may be embodied as one element or one `portion, module, member, or block' may include a plurality of elements.

Throughout the specification, when it is stated that one part is "connected to" another part, the part may be directly or indirectly connected to the other part and the indirect connection includes connection through a wireless communication network.

Also, when it is stated that a part "includes" an element, unless particularly defined otherwise, it means that the part does not exclude other elements and may further include other elements.

Through out the specification, when it is stated that one member is positioned "on" another member, not only the one member may be in contact with the other member but also another member may be present between the two members.

The terms fist, second, and the like are used for distinguishing one element from other elements but not intended to limit the elements.

Singular expressions, unless contextually otherwise defined, include plural expressions. In each of operations, reference numbers are used for convenience of description but not intended
to describe order of each of operations. Each of operations may be performed unlike a stated order unless definitely defined by context.

Hereinafter, operational principles and embodiments of the present invention will be described with reference to the attached drawings.

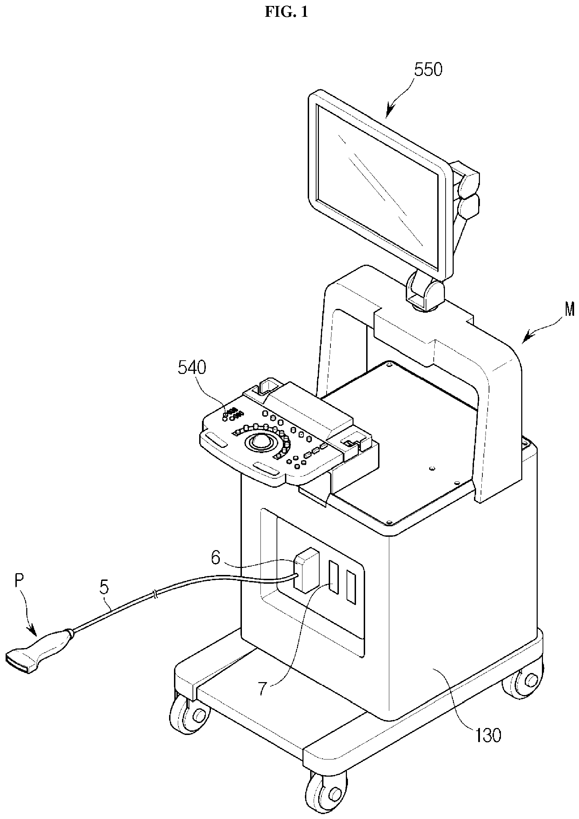

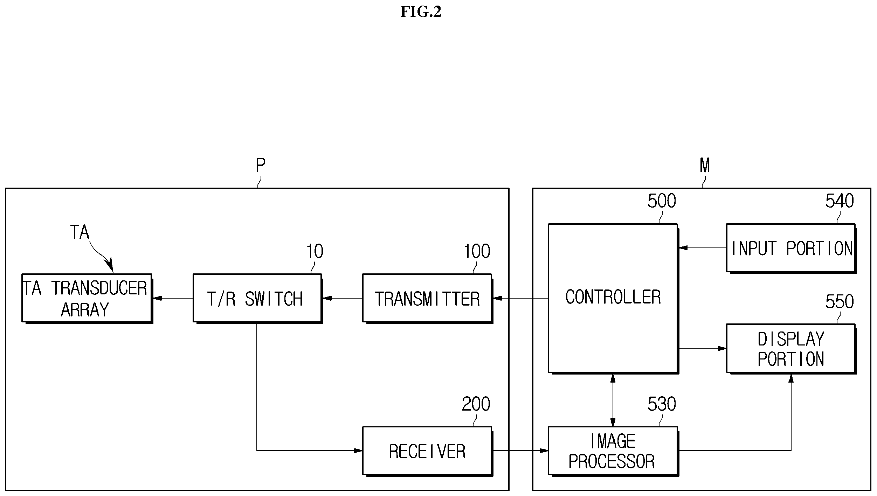

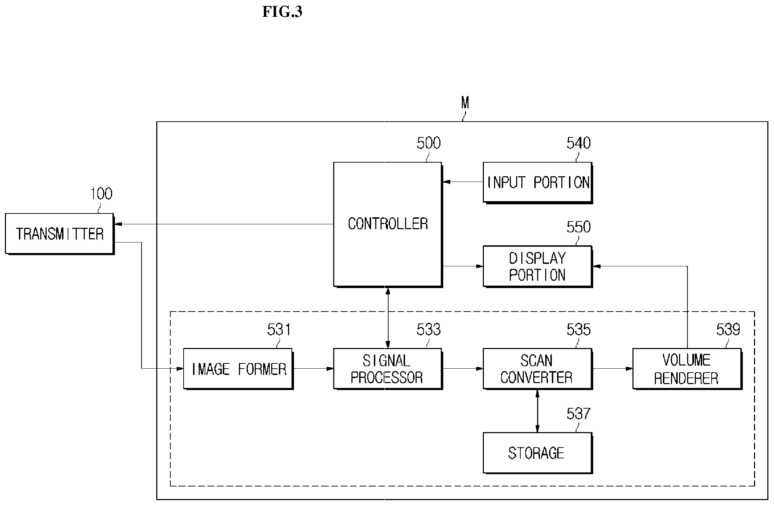

FIG. 1 is an external view of an ultrasound imaging apparatus in accordance with one embodiment, and FIG. 2 is a control block diagram of the ultrasound imaging apparatus in accordance with one embodiment. Also, FIG. 3 is a control block diagram illustrating a configuration of a main body of the ultrasound imaging apparatus in accordance with one embodiment.

Referring to FIG. 1, an ultrasound imaging apparatus 1 includes an ultrasound probe P configured to transmit ultrasonic waves to an object, receive ultrasonic echo signals from the object, and convert the ultrasonic echo signals into electrical signals and a main body M connected to the ultrasound probe P and configured to include an input portion 540 and a display portion 550 and display an ultrasonic image. The ultrasound probe P may be connected to the main body M of the ultrasound imaging apparatus 1 through a cable 5, may receive various signals necessary for controlling the ultrasound probe P, and may transfer analog signals or digital signals corresponding to the ultrasonic echo signals received by the ultrasound probe P to the main body M. However, embodiments of the ultrasound probe P are not limited thereto and the ultrasound probe P may be embodied as a wireless probe to transmit and receive signals through a network formed between the ultrasound probe P and the main body M.

One end of the cable 5 may be connected to the ultrasound probe P and the other end thereof may be connected to a connector 6 configured to be couplable with or separable from a slot 7 of the main body M. The main body M and the ultrasound probe P may send and receive control commands or data using the cable 5. For example, when a user inputs information with respect to a focal depth, a size or shape of aperture, a steering angle or the like through the input portion 540, such pieces of information may be transferred to the ultrasound probe P through the cable 5 to be used for forming beams transmitted and received between a transmitter 100 and a receiver 200. Also, when the ultrasound probe P is embodied as a wireless probe as described above, the ultrasound probe P is connected to the main body M through a wireless network not the cable 5.

When connected to the main body M through a wireless network, the main body M and the ultrasound probe P may send and receive the control commands or data described above. The main body M may include a controller 500, an image processor 530, the input portion 540, and the display portion 550 as shown in FIG. 2.

The controller 500 controls overall operations of the ultrasound imaging apparatus 1. In detail, the controller 500 generates control signals for controlling each of elements of the ultrasound imaging apparatus 1 such as the transmitter 100, a T/R switch 10, the receiver 200, the image processor 530, and the display portion 550 as shown in FIG. 2 and controls operations of all the elements described above. In the ultrasound imaging apparatus 1 according to the embodiment shown in FIGS. 2 and 3, a transmitting/receiving beamformer is included in the ultrasound probe P not the main body M. However, the transmitting/receiving beamformer may be included in the main body M not the ultrasound probe P.

The controller 500 calculates a delay profile of a plurality of ultrasonic transducer elements 60 that form an ultrasonic transducer array TA and calculates a time delay value according to a distance difference of a focal point between each of the plurality of ultrasonic transducer elements 60 included in the ultrasonic transducer array TA and the object based on the calculated delay profile. Also, the controller 500 controls the transmitting/receiving beamformer according thereto to generate transmission/reception signals.

Also, the controller 500 may control the ultrasound imaging apparatus 1 by generating a control command for each of the elements of the ultrasound imaging apparatus 1 according to an instruction or command of a user input through the input portion 540.

The controller 500 according to the disclosed embodiment sets a region of interest (ROI) in an ultrasonic image, particularly, in a sonoelastographic image displayed on the display portion 550 as a shape corresponding to that of an interested object not a simple circular or quadrangular shape including the interested object. For example, a shape corresponding to a cardiac short axis plane that is an interested object in an ultrasonic image of the heart is set as an ROI and the region of interest is displayed on the display portion 550. A detailed description thereof will be described below.

The image processor 530 generates an ultrasonic image of a target part inside the object based on ultrasonic signals collected by the receiver 200.

The ultrasound imaging apparatus 1 may be used for inspecting a heart. It is referred to as an echocardiography that forms an image by sending ultrasonic waves to a heart and analyzing the ultrasonic waves that are reflected and return and estimates a morphological structure and function of the heart.

The echocardiography has a difference from a general ultrasound exam in an aspect of inspecting a moving structure that is a heart.

Also, unlike general ultrasound exams, the echocardiography diagnoses a cardiac function by measuring the capacity, pressure and the like in the heart using a Doppler and the like.

In detail, sizes of atria and ventricles of the heart may be measured and a function as a pump for the heart and a function of receiving blood into the heart are evaluated. Also, since the thickness of a cardiac wall may be directly measured, myocardiopathy and the like may be easily diagnosed and a survival rate and prognosis of a patient may be estimated. Since the echocardiography provides a shape of a valve in the heart and hemodynamic information, a cause and a degree of a valvular disease may be estimated using only an ultrasound exam. For example, it is possible to check a movement disorder of a local cardiac wall of a heart in case of ischemic heart diseases, and more particularly, it is possible to do a differential diagnosis of myocardial infraction with a high mortality through an echocardiography.

Referring to FIG. 3, the image processor 530 may include an image former 531, a signal processor 533, a scan converter 535, a storage 537, and a volume renderer 539.

The image former 531 generates a two-dimensional or three dimensional coherent ultrasonic image of a target part inside an object based on ultrasonic signals collected by the receiver 200.

The signal processor 533 converts coherent image information formed by the image former 531 into ultrasonic image information according to a diagnosis mode such as a B-mode, a Doppler mode or the like. For example, when a diagnosis mode is set as a B-mode, the signal processor 533 performs processing such as an A/D conversion treatment and compiles ultrasonic image information for a B-mode image in real time. Also, when a diagnosis mode is set as a Doppler mode (D-mode), the signal processor 533 extracts phase shift information from an ultrasonic signal, calculates information such as a blood current corresponding to each point of a photographed cross section such as a velocity, power, and dispersion, and compiles ultrasonic image information of the D-mode image in real time.

Also, when a diagnosis mode is set as an elastic mode, the signal processor 533 calculates a disparity image from consecutive ultrasonic images, detects a point at which a disparity of tissue occurs, and calculates a velocity of the disparity. In this case, an alteration degree of a scatter is calculated by comparing the consecutive ultrasonic images to detect the disparity of tissue.

Sequentially, the signal processor 533 measures a transmission velocity of a transverse wave by determining a position of the transverse wave in an image from each disparity image and calculates a shear modulus based on the transmission velocity of the transverse wave. The shear modulus is calculated by multiplying a square of the transverse wave by a density of a medium.

The signal processor 533 calculates the shear modulus as a grade of elasticity and generates a sonoelastographic image based on the calculated the grade of elasticity. The sonoelastographic image may include a preset color according to a grade of elasticity of a region of interest and may be embodied as a spectrum image displayed as a three-dimensional image or a waveform. The sonoelastographic image according to the disclosed embodiment may be generated using external pressure or may be generated using internal pressure, for example, pressure generated from a cardiac vessel.

The scan converter 535 converts converted ultrasonic image information input from the signal processor 533 or converted ultrasonic image information stored in the storage 537 into a general video signal for the display portion 550 and transmits the video signal to the volume renderer 539.

The storage 537 temporarily or non-temporarily stores the ultrasonic image information converted through the signal processor 533.

The volume renderer 539 performs volume rendering based on the video signal transmitted from the scan converter 535, generates a final result image by correcting rendered image information, and transmits the generated result image to the display portion 550. For example, the volume renderer 539 may perform the volume rendering of the heart based on the video signal of the heart transmitted from the scan converter 535.

The input portion 540 may be provided to allow a user to input commands related to operations of the ultrasound imaging apparatus 1. The user may input or set diagnosis mode selection commands such as an ultrasonic diagnosis starting command, a brightness mode (B-mode), a motion mode (M-mode), a Doppler mode (D-mode), an elastic mode, a three-dimensional mode and the like and ROI setting information including a size and a position of an ROI. The ROI may be at least one of a long axis cross section and a short axis cross section of the heart but is not limited thereto. A detailed description thereof will be described below.

The B-mode is displaying an internal cross-sectional image of an object and illustrates a part with a strong reflected echo and a part with a weak reflected echo using a difference in brightness. A B-mode image is configured based on information obtained from several tens to several hundreds of scan lines.

The M-mode is displaying how biometric data (for example, brightness information) with respect to a certain part (M line) among cross-sectional images (B-mode images) of the object changes according to time as an image. Generally, the B-mode image and the M-mode image are displayed in one screen at the same time to allow a user to make an accurate diagnosis by comparing and analyzing two data.

The D-mode means an image using the Doppler effect in which a frequency of a sound emitted from a moving object causes a change. The mode using the Doppler effect described above may be subdivided into a power Dopper imaging (PDI) mode, a color flow mode (S flow), and a DPDI mode.

The PDI mode is displaying the degree of a Doppler signal or the number of a structure (red blood cells in blood) and is less sensitive to an incident angle in such a way that there is no fake signal and an image is less attenuated. Also, since the PDI mode records reflected Doppler energy, the PDI mode is very sensitive to even detect a small blood vessel and a blood flow at a low velocity.

The color flow mode (S flow) provides a PDI that shows power of a Doppler signal in a two-dimensional distribution and a velocity image that indicates a velocity of a Doppler signal in a two-dimensional distribution. A color flow mode image may not only visualize a blood flow in real time but also show a widespread blood flow state from a high velocity blood flow in a large blood vessel to a low velocity blood flow in a small blood vessel.

The DPDI mode means a directional image that shows directional information of a Doppler signal in the PDI mode in a two-dimensional distribution. Accordingly, there is an effect of more accurately detecting information with respect to a blood flow than the PDI. Also, an M-mode image may be generated with respect to a Doppler mode image.

The elastic mode means a method of obtaining a sonoelastographic image of an object using elastography. Here, the elastography indicates analyzing that a difference in metamorphic grades of tissue according to pressure is decreased because the harder structure such as a malignant mass has lower tissue elasticity. The sonoelastographic image means an image that quantitatively displays stiffness of tissue as described above.

The three-dimensional mode generally means an image that displays a geometric three-dimensional structure or space including X, Y, and Z values that represent a depth, area, and height and may mean a series of images that are three-dimensional shapes or mean a three-dimensional effect or stereo effect. As an example, a shape of a heart may be three-dimensionally displayed using a stereo effect of the three-dimensional mode.

The input portion 540 may include various devices that allow a user to input an instruction or command such as a keyboard, a mouse, a trackball, a tablet, a touch screen module and the like.

Particularly, the user may input a region of interest that is desired by the user through the input portion 540.

The display portion 550 displays a menu or guidance necessary for echography and an ultrasonic image obtained during an echography process. The display portion 550 displays an ultrasonic image of a target part inside an object generated by the image processor 530. An ultrasonic image displayed on the display portion 550 may be an ultrasonic image of the B-mode, an ultrasonic image in the elastic mode, and a three-dimensional ultrasonic image. The display portion 550 may display various ultrasonic images according to the modes described above. When displaying a sonoelastographic image, the display portion 550 may display a color preset according to a grade of elasticity (that is, a shear modulus) of each point. Also, the display portion 550 may display a grade of elasticity of each point in a region of interest that is digitized.

The display portion 550 may be embodied in various well-known displaying devices such as a cathode ray tube (CRT), a liquid crystal display (LCD) and the like.

The ultrasound probe P according to one embodiment, as shown in FIG. 2, may include the ultrasonic transducer array TA, the T/R switch 10, the transmitter 100, and the receiver 200. The ultrasonic transducer array TA may be provided at an end portion of the ultrasound probe P. The ultrasonic transducer array TA means arranging a plurality of ultrasonic transducer elements 60 in one-dimensional or two-dimensional array. The ultrasonic transducer array TA generates ultrasonic waves while vibrating due to an applied pulse signal or an alternating current. The generated ultrasonic waves are transmitted to a target part inside an object. In this case, the ultrasonic waves generated at the ultrasonic transducer array TA may be transmitted with a plurality of target parts inside the object as focuses. In other words, the generated ultrasonic waves may be transmitted to the plurality of target parts while being multi-focused.

The ultrasonic waves generated at the ultrasonic transducer array TA are reflected by the target parts inside the object and return to the ultrasonic transducer array TA. The ultrasonic transducer array TA receives ultrasonic echo signals that are reflected by the target parts and return therefrom. When the ultrasonic echo signals arrive, the ultrasonic transducer array TA vibrates at a certain frequency corresponding to a frequency of the ultrasonic echo signals and outputs an alternating current at a frequency corresponding to a vibrating frequency. Accordingly, the ultrasonic transducer array TA converts the received ultrasonic echo signals into certain electric signals. Since each of the ultrasonic transducer elements 60 receives an ultrasonic echo signal and outputs an electrical signal, the ultrasonic transducer array TA may output electrical signals in a plurality of channels.

An ultrasonic transducer may be embodied as any one of a magnetostrictive ultrasonic transducer using a magnetostrictive effect of a magnetic body, a piezoelectric transducer using a piezoelectric effect of a piezoelectric material, and a capacitive micromachined ultrasonic transducer (CMUT) that transmits and receives ultrasonic waves using vibrations of several hundreds or several thousands of micromachined thin films. Also, in addition thereto, other types of transducers capable of generating ultrasonic waves or generating electrical signals according to ultrasonic waves may also be examples of the ultrasonic transducer.

For example, the ultrasonic transducer element 60 according to the disclosed embodiment may include a piezoelectric vibrator or a thin film. When an alternating current is applied from a power source, the piezoelectric vibrator or the thin film vibrates at a certain frequency according to the alternating current and generates ultrasonic waves at a certain frequency according to the vibrating frequency. On the other hand, when an ultrasonic echo signal at a certain frequency arrives at the piezoelectric vibrator or the thin film, the piezoelectric vibrator or the thin film vibrates according to the ultrasonic echo signal and outputs an alternating current at a frequency corresponding to the vibrating frequency.

The transmitter 100 applies a transmission pulse to the ultrasonic transducer array TA to allow the ultrasonic transducer array TA to transmit an ultrasonic signal to a target part inside an object. The transmitter 100 may include a transmission beamformer 110 and a pulser 120.

The transmission beamformer 110 forms a transmission signal pattern according to a control signal of the controller 500 of the main body M and outputs the transmission signal pattern to the pulser 120. The transmission beamformer 110 forms a transmission signal pattern based on a time delay value with respect to each of the ultrasonic transducer elements 60 that form the ultrasonic transducer array TA calculated through the controller 500 and transmits the formed transmission signal pattern to the pulser 120.

The receiver 200 performs a certain process with respect to the ultrasonic echo signal received from the ultrasonic transducer array TA and performs reception beamforming. The receiver 200 may include a reception signal processor and a reception beamformer. An electrical signal converted by the ultrasonic transducer array TA is input to the reception signal processor.

The reception signal processor may amply a signal before signal-processing or time delay processing with respect to the electrical signal obtained by converting the ultrasonic echo signal and may adjust a gain or compensate a decrease according to a depth. In more detail, the reception signal processor may include a low noise amplifier (LNA) that decreases noise with respect to the electrical signal input from the ultrasonic transducer array TA and a variable gain amplifier (VGA) that controls a gain value according to the input signal. The variable gain amplifier may perform time gain compensation (TGC) for compensating a gain according to a distance from a focusing point but is not limited thereto.

The reception beamformer performs beamforming with respect to the electrical signal input from the reception signal processor. The reception beamformer increases signal strength of the electrical signal input from the reception signal processor through superposition. The signal beamformed by the reception beamformer is converted into a digital signal while passing through an analog-to-digital converter and is transmitted to the image processor 530 of the main body M.

When the analog-to-digital converter is provided at the main body M, an analog signal beamformed by the reception beamformer may be transmitted to the main body M and may be converted into a digital signal at the main body M. Also, the reception beamformer may be a digital beamformer. The digital beamformer may include a storage portion capable of sampling and storing an analog signal, a sampling period controller capable of controlling a sampling period, an amplifier capable of adjusting amplitude of a sample, an anti-aliasing low pass filter for preventing aliasing before sampling, a bandpass filter capable of selecting a desired frequency band, an interpolation filter capable of increasing a sampling rate in beamforming, and a high-pass filter capable of removing a digital current (DC) component or a signal in a low frequency band.

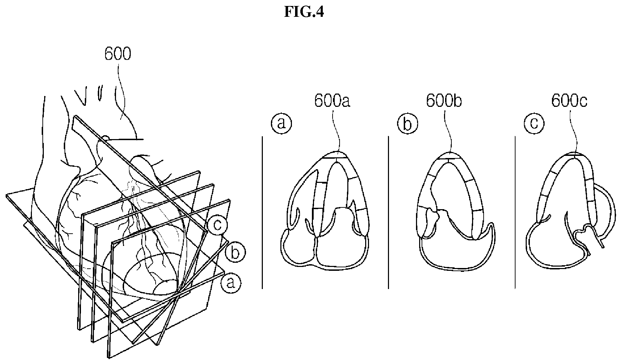

FIG. 4 is a view illustrating a long axis cross section of a heart.

Referring to FIG. 4, FIG. 4 illustrates a cross section position 600 and cross sections 600a, 600b, and 600c of the heart. In analyzing of the heart, the ultrasonic image apparatus may form a cross section based on a long axis.

A normal 4 chamber plane (A4C) 600a is a cross section of A plane of the heart and is a plane in which all of a left ventricle, a left atrium, a right ventricle, and a left atrium are shown. In the A4C 600a, an apical cap, an apical septum, a mid inferoseptum, a basal inferoseptum, an apical lateral, a mid anterolateral, and a basal anterolateral are shown.

The A4C 600a is a cross-sectional view of the heart longitudinally taken from the left ventricle. Through the A4C 600a, it is possible to most easily observe a mitral valve and a ventricular septum. Through the A4C 600a, the left and right atria and ventricles, the mitral valve and a tricuspid valve between each atrium and each ventricle, and an atrial septum and the ventricular septum are shown.

Meanwhile, a normal 2 chamber plane (A2C) 600b is a cross section of B plane of the heart and is a plane in which atria and ventricles are shown. In the A2C 600b, an apical cap, an apical inferior, a mid interior, and a basal inferior are shown. Also, an apical anterior, a mid anterior, and a basal anterior are shown.

The A2C 600b is obtained by rotating a long axis tomogram of the left ventricle by 90 degrees and is obtained by rotating the A4C 600a by 60 degrees counterclockwise to allow the left atrium, the left ventricle, and the mitral valve to be seen. In color Doppler echocardiogram, since it is easy to detect countercurrents of AR and MR, the A2C 600b may be used. Also, since it is possible to obtain information with respect to an end portion of the heart at the left ventricle, the A2C 600b is a cross section necessary for ischemic heart diseases.

A three chamber plane (A3C) 600c is obtained by rotating the A2C 600b by 60 degrees counterclockwise to allow the left atrium, the left ventricle, and an aortic valve including a left ventricular outflow path to be seen.

The A3C 600c is a cross section of C plane of the heart and is a plane in which three chambers are shown. In the A3C, the left ventricle, the left atrium, and an aorta are shown. In the A3C, an apical lateral, a mid inferolateral, a basal inferolateral, an apical anterior, a mid anteroseptum, and a basal anteroseptum are shown.

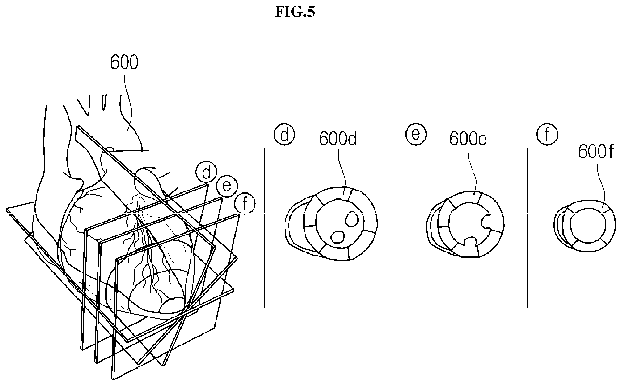

FIG. 5 is a view illustrating a short axis cross section of a heart.

Referring to FIG. 5, FIG. 5 illustrates a cross section position 600 and cross sections 600d, 600e, and 600f of the heart. In analyzing of the heart, the ultrasonic image apparatus may form a cross section based on a short axis.

A short axis basic plane (SAXB) 600d is a cross section of D plane of the heart. In the SAXB 600d, an anterior, an anteroseptum, an inferoseptum, an anterior lateral, an inferolateral, and an inferior of the heart are shown.

A short axis mid plane (SAXM) 600e is a cross section of E plane of the heart. The SAXM 600e illustrates an internal structure of the heart unlike the SAXB 600d, and particularly, shows different shapes of the heart at the inferior and anterior laterals of the heart.

A short axis normal plane (SAXA) 600f is a cross section of F plane of the heart. The SAXA 600f shows an anterior, a septum, a lateral, and an inferior.

A cross section based on a short axis of the heart may show a mitral valve (atrioventicular valve) and a left mitral valve and has an effect on inspecting whether valve calcification is present and inspecting an area of an inlet portion of a valve.

Accordingly, four chambers, that is, left and right ventricles and left and right atria are illustrated as the A4C view. In the A3C view, the left ventricle, the left atrium, and the aorta are shown. Also, when a heart is three-dimensionally shown, the views may be reconfigured as multiplanar reformat/reconfiguration (MPR) planes. Detecting of two-dimensional planes in a 3D volume may improve consistency among users and may be used for adjusting parameters obtained for excellent image quality.

That is, the controller 500 may obtain the A4C, the A2C, the A3C, the SAXB, the SAXM, and the SAXA, and the cross sections described above may allow a treatment operation flow and a movement analysis of internal walls of the heart to be easily performed.

In FIGS. 4 and 5, the cross sections of the heart have been described. However, cross sections capable of being displayed by the ultrasonic image apparatus are not limited thereto and the cross sections described above are merely examples of the cross sections of the heart.

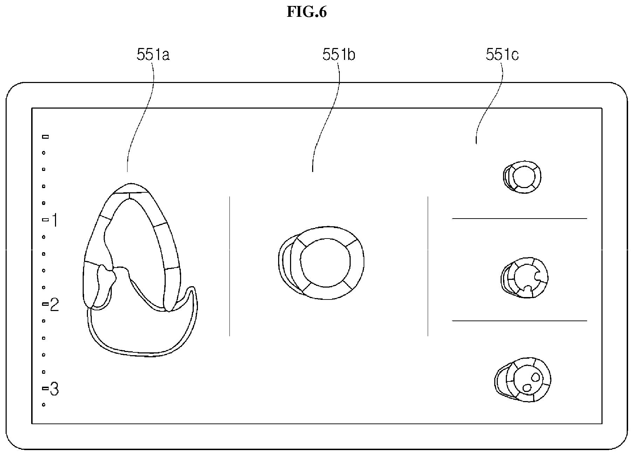

FIG. 6 is a view illustrating a long axis cross section and a short axis cross section of a heart that are illustrated on a display according to the disclosed embodiment.

Referring to FIG. 6, the controller 500 may obtain a long axis cross section 551a of a heart and may control the long axis cross section 551a to be shown on one side of a display. In FIG. 6, the A2C is shown. However, the long axis cross section 551a of the heart shown on a display by the controller 500 may be the A4C and the A3C described above but is not limited in type.

Meanwhile, the user may set an ROI through the input portion 540. The controller 500 may show the long axis cross section 551a and a short axis cross section 551b of the heart shown in advance based on the ROI set by the user. In FIG. 6, even though the user sets an SAXA as the ROI, an SAXB and an SAXM may be set as the ROI. However, the ROI set by the user is not limited thereto.

Meanwhile, when the long axis cross section 551a is shown on the display and the user selects any one of short axis cross sections as the ROI, the controller 500 may control a list 551c of short axis cross sections of the heart to be output on the one side of the display. The display may output the list 551c of the short axis cross sections of the heart on the one side.

The long axis cross section 551a and the short axis cross section 551b of the heart may be output on one screen at the same time to allow the user to accurately and quickly recognize a cardiac structure.

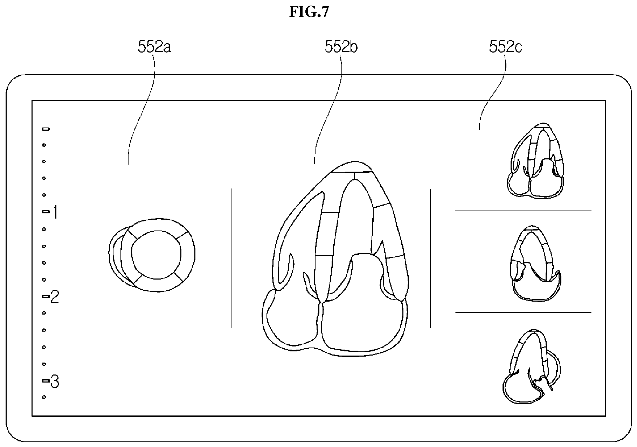

FIG. 7 is a view illustrating a short axis cross section 552a and a long axis cross section 552b of a heart that are illustrated on a display according to the disclosed embodiment.

Referring to FIG. 7, the controller 500 may obtain the short axis cross section 552a of the heart and may control the short axis cross section 552a to be shown on one side of a display. In FIG. 7, a short axis normal plane is shown. However, the short axis cross section 552a of the heart shown on the display portion 550 by the controller 500 may be a short axis basic plane and a short axis mid plane but is not limited in type thereof.

Meanwhile, the user may set an ROI through the input portion 540. The controller 500 may show the short axis cross section 552a and the long axis cross section 552b of the heart shown in advance based on the ROI set by the user. In FIG. 7, even though the user sets an A4C as the ROI, an A2C and an A3C may be set as the ROI. However, the ROI set by the user is not limited thereto.

Meanwhile, when the short axis cross section 552a is shown on the display and the user selects any one of long axis cross sections as the ROI, the controller 500 may control a list 552c of long axis cross sections of the heart to be output on one side of the display portion 550. The display portion 550 may output the list 552c of the long axis cross sections of the heart on the one side.

The short axis cross section 552a and the long axis cross section 552b of the heart may be output on one screen at the same time to allow the user to accurately and quickly recognize a cardiac structure.

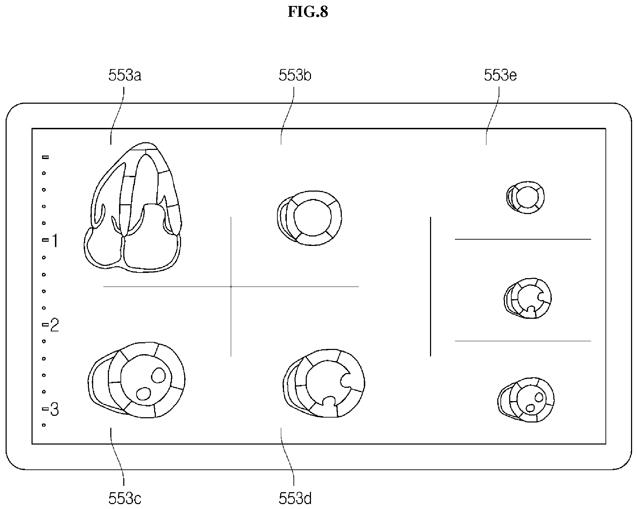

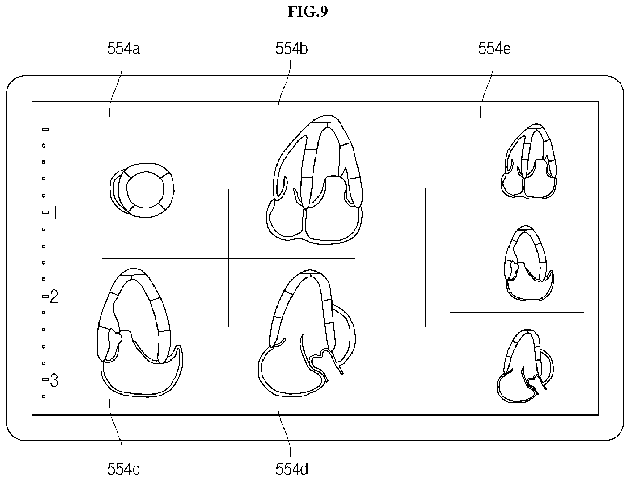

FIG. 8 is a view illustrating one long axis cross section and three short axis cross sections of a heart displayed on the display portion 550, and FIG. 9 is a view illustrating one short axis cross section and three long axis cross sections of a heart displayed on one display portion 550. Referring to FIG. 8, FIG. 8 illustrates that a long axis cross section 553a and three short axis cross sections 553b, 553c, and 553d are displayed on one display portion 550. In FIG. 8, an A4C is shown. However, both an A2C and an A3C described above are available.

Referring to FIG. 9, FIG. 9 illustrates that a short axis cross section 554a and three long axis cross sections 554b, 554c, and 554d are displayed on one display portion 550. In FIG. 9, an SAXA is displayed. However, both an SAXB and an SAXM are available.

The user may easily and quickly recognize a cardiac structure by comparing one cardiac long axis plane or one short axis plane with several cardiac short axis planes or long axis planes.

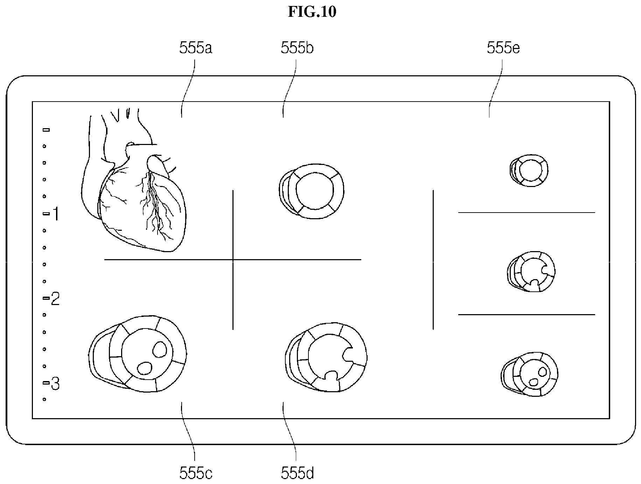

FIG. 10 is a view illustrating that one stereoradiographic image and three short axis cross sections of a heart according to the disclosed embodiment are displayed on the display portion 550.

The controller 500 may derive a stereoscopic image 555a based on an image obtained by the ultrasound probe P and may control the display portion 550 to display the stereoscopic image 555a. Since an operation of obtaining a stereoscopic image of a heart has been described above, a detailed description thereof will be omitted.

Referring to FIG. 10, FIG. 10 illustrates that the stereoscopic image 555a and short axis cross sections 555b, 555c, and 555d of the heart are displayed on one display portion 550. The user may set an ROI through the input portion 540 while observing a stereoscopic image of a heart. The user may set a cardiac short axis cross section as the ROI or may set a cardiac long axis cross section as the ROI. In FIG. 10, the short axis cross sections 555b, 555c, and 555d of the heart are set as the ROI and three short axis cross sections of the heart are shown. Even though the short axis cross sections of the heart are set as the ROI as shown in FIG. 10, the user may set the long axis cross section of the heart as the ROI. In this case, an A4C, an A2C, and an A3C may be shown with the stereoscopic image of the heart.

Meanwhile, the controller 500 may adjust an angle of the stereoscopic image 555a of the heart. The user may input a signal for changing the angle of the stereoscopic image 555a of the heart through the input portion 540, and the controller 500 may change the angle of the stereoscopic image 555a of the heart based on the signal input by the user. The controller 500 may output images of the short axis cross sections 555b, 555c, and 555d based on the stereoscopic image of the heart at the changed angle.

Since it is possible to observe a stereoscopic image of a heart and short axis cross sections of the heart through one display portion 550, the user may more efficiently recognize a cardiac structure.

Even though FIGS. 8 to 10 illustrate images obtained by dividing a part in which an image of a heart is displayed into four parts, they are merely one embodiment and there is no limitation in position and number of the images of the heart.

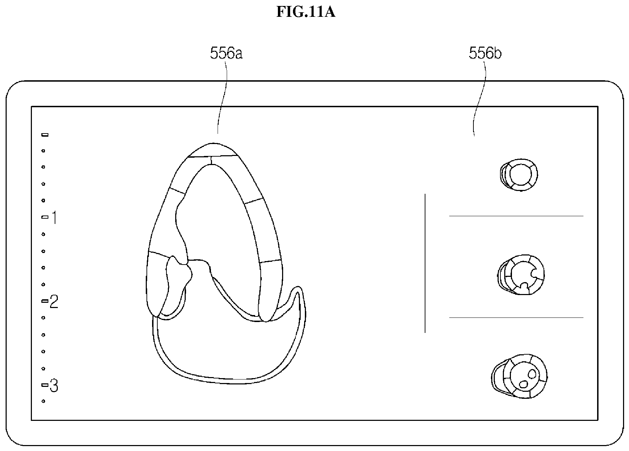

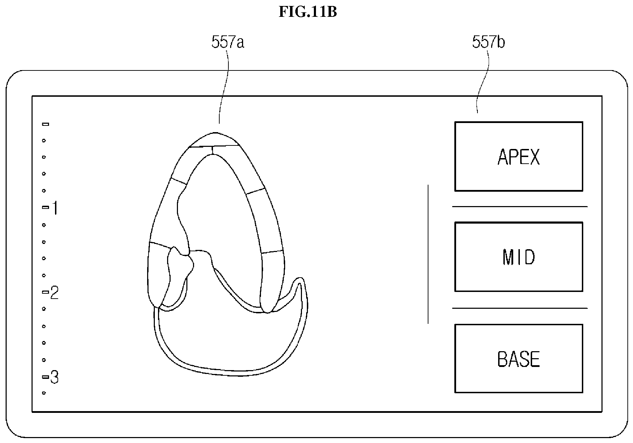

FIGS. 11A and 11B are views illustrating interfaces for inputting an ROI according to the disclosed embodiment.

Referring to FIG. 11A, FIG. 11A illustrates an A2C 556a of long axis cross sections of a heart and three short axis cross sections 556b of the heart. The user may compare two images through a command for selecting one of the short axis cross sections of the heart displayed on the display portion 550.

Referring to FIG. 11B, FIG. 11B displays an A2C 557a of the cardiac long axis cross sections. In FIG. 11B, not the short axis cross section of the heart but names 557b of cross sections are shown. The user may select at least one of the name of cross sections and may compare a long axis cross section image of the heart with a short axis cross section image of the heart.

As described above, a command for inputting the ROI in FIGS. 11A and 11B may be input through the input portion 540. Otherwise, when the display portion 550 includes a touch panel, a command may be input through a touch to the display portion 550.

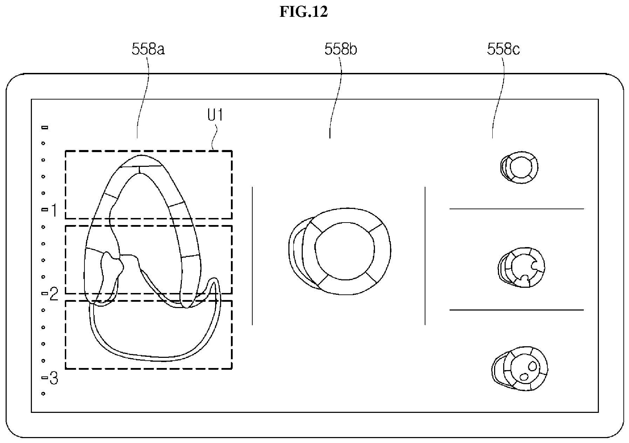

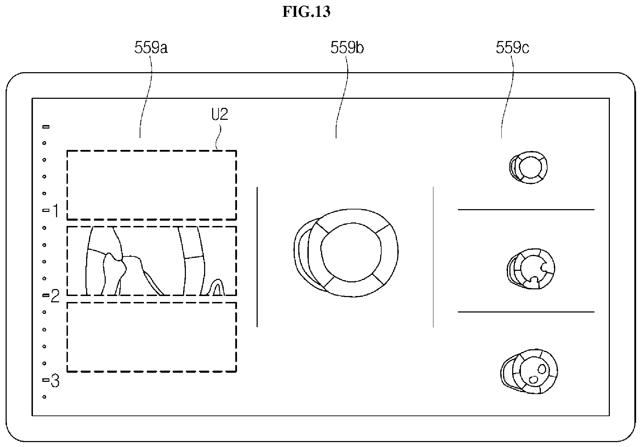

FIGS. 12 and 13 are views illustrating ROIs according to the disclosed embodiment.

Referring to FIG. 12, the controller 500 may display a guide line U1 of an ROI on a long axis cross section 558a of a heart output on the display portion 550. The controller 500 may make structural division of the long axis cross section 558a to be precise by displaying the long axis cross section 558a of the heart with the guide line U1 of the ROI and may induce the ROI of an area desired by the user in more detail.

In FIG. 12, the A2C 558a of long axis cross sections of the heart is shown and an area U1 of a short axis normal plane, a short axis basic plane, and a short axis mid plane is displayed in the A2C 558a. The user may select the ROI through the input portion 540. In FIG. 12, a short axis mid plane 558b is selected and the A2C 558a and the short axis mid plane 558b are shown together.

Referring to FIG. 13, FIG. 13 illustrates that the user selects a short axis mid plane 559b as an ROI through the input portion 540 in an A4C 559a.

The controller 500 may display a cardiac long axis cross section 559a that displays only an ROI U2 set by the user in the long axis cross section 559a and may display a cardiac short axis cross section 559b corresponding thereto. The cardiac long axis cross section 559a shown in FIG. 12 is an A2C, and the ROI selected by the user is the short axis mid plane 559b. Through operations described above, the user may perform more intensive observation by observing the cardiac long axis cross section of a part U2 to be observed while separating from a part not to be observed.

Types of cross sections shown in FIGS. 12 and 13 are merely one embodiment, and types of cross sections to be displayed with the guide line of the ROI are not limited.

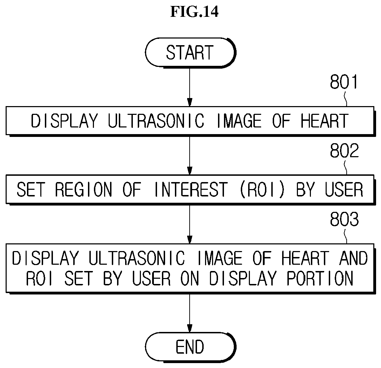

FIG. 14 is a flowchart according to the disclosed embodiment.

Referring to FIG. 14, the ultrasound imaging apparatus 1 may display an ultrasonic image of a heart (801). An existing cross section may be a cardiac long axis cross section or a cardiac short axis cross section. As described above, the long axis cross section of the heart may be an A4C, an A2C, and an A3C and the cardiac short axis cross section may be a short axis normal plane, a short axis mid plane, and a short axis basic plane. The user may set an ROI in the existing cross section (802). The controller 500 displays a cardiac cross section of the ROI set by the user and an existing ultrasonic image of the heart on one display portion 550 (803). Through this, the user may more effectively and quickly recognize a cardiac structure than existing displaying methods.

The ROI setting method described above may be applied to a three-dimensional ultrasonic image. The user may adjust a shape or a size of an ROI displayed on the display portion 550 through inputting of a command through the input portion 540 or inputting of a command through a touch to the display portion 550.

Meanwhile, the disclosed embodiments may be embodied as recording media that store commands executable by computers. The commands may be stored as the form of program codes and may generate program modules when being executed by a processor to perform operations of the disclosed embodiments. The recording media may be embodied as computer-readable recording media.

The computer-readable recording media may include all types of recording media that store computer-decodable commands. For example, there may be a read only memory (ROM), a random access memory (RAM), a magnetic tape, a magnetic disc, a flash memory, an optical data storage and the like.

As is apparent from the above description, an ultrasound imaging apparatus and a method of controlling the same in accordance with one embodiment of the present disclosure may more efficiently and quickly recognize a cardiac structure by displaying a cardiac image obtained by the ultrasound imagining apparatus and an image of an interested cardiac area set by a user at the same time.

Although a few embodiments of the present disclosure have been shown and described, it would be appreciated by those skilled in the art that changes may be made in these embodiments without departing from the principles of the present disclosure, the scope of which is defined in the claims .

## Claims

1. An ultrasound imaging apparatus (1) comprising:
a controller (500) configured to obtain an ultrasonic image of the heart at different angles and to derive a stereoscopic image (555a);
a display portion (550) configured to display the ultrasonic image of the heart of an object, wherein the ultrasonic image is the stereoscopic image (555a) that three-dimensionally shows the heart;
an input portion (540) configured to receive a command for setting a region of interest (ROI) of the displayed ultrasonic image of the heart;
the controller (500) being further configured to set the ROI of the ultrasonic image of the heart based on the command for setting the ROI input through the input portion and control at least one image of the set ROI and the ultrasonic image of the heart to be displayed together on the display portion,
wherein the controller (500) divides the display portion (550) into at least two areas and controls the ultrasonic image of the heart and the at least one ROI image to be displayed in the divided areas, respectively.

2. The ultrasound imaging apparatus (1) of claim 1, wherein the ultrasonic image of the heart is one of cardiac long axis cross sections (551a, 553a, 556a), of the heart, and
wherein the ROI image is at least one of cardiac short axis cross sections (551b; 553b, 553c, 553d; 556b) of the heart.

3. The ultrasound imaging apparatus (1) of claim 1, wherein the ultrasonic image of the heart is one of cardiac short axis cross sections (552a; 554a) of the heart, and
wherein the ROI image is at least one of cardiac long axis cross sections (552b; 554b, 554c, 554d) of the heart.

4. The ultrasound imaging apparatus (1) of claim 1,
wherein the ROI image is at least one of a short axis cross section image (555b, 555c, 555d) and a long axis cross section image of the heart.

5. The ultrasound imaging apparatus (1) of claim 1, wherein the controller (500) controls the ultrasonic image of the heart and a guide line (U1) related to setting of the ROI in the ultrasonic image to be displayed together.

6. The ultrasound imaging apparatus (1) of claim 1, wherein when the ROI of the ultrasonic image of the heart is selected through the input portion (540), the controller (500) controls only an ultrasonic image of an area corresponding to the ROI selected from the ultrasonic image of the heart to be displayed.

7. The ultrasound imaging apparatus (1) of claim 1, wherein the display portion (550) comprises a touch panel configured to receive a command for selecting the ROI of the ultrasonic image of the heart, and
wherein when the ROI of the ultrasonic image of the heart is selected through the display portion (550), the controller (500) controls the ultrasonic image of the heart and the ROI image to be displayed on the display portion (550).

8. The ultrasound imaging apparatus (1) of claim 1, wherein the controller (500) obtains and displays a list (551c, 552c) of a plurality of such preset ROI images and controls at least one ROI image selected from the list of the plurality of displayed ROI images to be displayed.

9. A method of controlling an ultrasound imaging apparatus (1) using a controller (500), comprising:
obtaining an ultrasonic image of the heart at different angles to derive a stereoscopic image (555a);
displaying on a display portion (550) the ultrasonic image of the heart of an object, wherein the ultrasonic image is the stereoscopic image (555a) that three-dimensionally shows the heart;
receiving on an input portion (540) a command for setting a region of interest (ROI) of the displayed ultrasonic image of the heart (802); and
setting the ROI of the ultrasonic image of the heart based on the command for setting the ROI input through an input portion (540) and controlling at least one image of the set ROI and the ultrasonic image of the heart to be displayed together (803),
wherein the controlling of at least one image of the set ROI and the ultrasonic image of the heart to be displayed together comprises controlling the ultrasonic image of the heart and the at least one ROI image to be displayed in divided areas, respectively.

10. The method of claim 9, wherein the ultrasonic image of the heart is one of cardiac long axis cross sections (551a, 553a, 556a) of the heart, and
wherein the ROI image is at least one of cardiac short axis cross sections (551b; 553b, 553c, 553d; 556b) of the heart.

11. The method of claim 9, wherein the ultrasonic image of the heart is one of cardiac short axis cross sections (552a, 554a) of the heart, and
wherein the ROI image is at least one of cardiac long axis cross sections (552b; 554b, 554c, 554d) of the heart.

12. The method of claim 9,
wherein the ROI image is at least one of a short axis cross section image (555b, 555c, 555d) and a long axis cross section image of the heart.

## Patentansprüche

1. Ultraschallbildgebungsvorrichtung (1), die Folgendes umfasst:
eine Steuerung (500), die so konfiguriert ist, dass sie ein Ultraschallbild des Herzens unter verschiedenen Winkeln erhält und ein stereoskopisches Bild (555a) ableitet;
einen Anzeigeabschnitt (550), der so konfiguriert ist, dass er das Ultraschallbild des Herzens eines Objekts anzeigt, wobei es sich bei dem Ultraschallbild um das stereoskopische Bild (555a) handelt, das das Herz dreidimensional darstellt;
einen Eingabeabschnitt (540), der so konfiguriert ist, dass er einen Befehl zum Einstellen einer Region von Interesse (ROI, Region Of Interest) des angezeigten Ultraschallbildes des Herzens empfängt;
wobei die Steuerung (500) ferner so konfiguriert ist, dass sie die ROI des Ultraschallbildes des Herzens basierend auf dem über den Eingabeabschnitt eingegebenen Befehl zum Einstellen der ROI einstellt und eine derartige Steuerung vornimmt, dass mindestens ein Bild der eingestellten ROI und das Ultraschallbild des Herzens zusammen auf dem Anzeigeabschnitt angezeigt werden,
wobei die Steuerung (500) den Anzeigeabschnitt (550) in mindestens zwei Bereiche unterteilt und eine derartige Steuerung vornimmt, dass das Ultraschallbild des Herzens und das mindestens eine ROI-Bild jeweils in den unterteilten Bereichen angezeigt werden.

2. Ultraschallbildgebungsvorrichtung (1) nach Anspruch 1, wobei es sich bei dem Ultraschallbild des Herzens um einen von Langachsenquerschnitten (551a, 553a, 556a) des Herzens handelt, und wobei es sich bei dem ROI-Bild um mindestens einen von Kurzachsenquerschnitten (551b; 553b, 553c, 553d; 556b) des Herzens handelt.

3. Ultraschallbildgebungsvorrichtung (1) nach Anspruch 1, wobei es sich bei dem Ultraschallbild des Herzens um einen von Kurzachsenquerschnitten (552a, 554a) des Herzens handelt, und
wobei es sich bei dem ROI-Bild um mindestens einen von Langachsenquerschnitten (552b; 554b, 554c, 554d) des Herzens handelt.

4. Ultraschallbildgebungsvorrichtung (1) nach Anspruch 1, wobei es sich bei dem ROI-Bild um mindestens eines von einem Kurzachsenquerschnittsbild (555b, 555c, 555d) und einem Langachsenquerschnittsbild des Herzens handelt.

5. Ultraschallbildgebungsvorrichtung (1) nach Anspruch 1, wobei die Steuerung (500) das Ultraschallbild des Herzens und eine Führungslinie (U1) in Bezug auf die Einstellung der ROI in dem Ultraschallbild so steuert, dass sie zusammen angezeigt werden.

6. Ultraschallbildgebungsvorrichtung (1) nach Anspruch 1, wobei, wenn die ROI des Ultraschallbildes des Herzens über den Eingabeabschnitt (540) ausgewählt wird, die Steuerung (500) eine derartige Steuerung vornimmt, dass nur ein Ultraschallbild eines Bereichs angezeigt wird, der der ROI entspricht, die aus dem Ultraschallbild des Herzens ausgewählt wurde.

7. Ultraschallbildgebungsvorrichtung (1) nach Anspruch 1, wobei der Anzeigeabschnitt (550) ein Berührungsfeld umfasst, das so konfiguriert ist, dass es einen Befehl zur Auswahl der ROI des Ultraschallbildes des Herzens empfängt, und
wobei, wenn die ROI des Ultraschallbildes des Herzens über den Anzeigeabschnitt (550) ausgewählt wird, die Steuerung (500) eine derartige Steuerung vornimmt, dass das Ultraschallbild des Herzens und das ROI-Bild auf dem Anzeigeabschnitt (550) angezeigt werden.

8. Ultraschallbildgebungsvorrichtung (1) nach Anspruch 1, wobei die Steuerung (500) eine Liste (551c, 552c) einer Vielzahl solcher voreingestellter ROI-Bilder erhält und anzeigt und eine derartige Steuerung vornimmt, dass mindestens ein ROI-Bild, das aus der Liste der Vielzahl angezeigter ROI-Bilder ausgewählt wird, angezeigt wird.

9. Verfahren zur Steuerung einer Ultraschallbildgebungsvorrichtung (1) unter Verwendung einer Steuerung (500), umfassend:
Erhalten eines Ultraschallbildes des Herzens unter verschiedenen Winkeln, um ein stereoskopisches Bild (555a) abzuleiten;
Anzeigen des Ultraschallbildes des Herzens eines Objekts auf einem Anzeigeabschnitt (550), wobei es sich bei dem Ultraschallbild um das stereoskopische Bild (555a) handelt, das das Herz dreidimensional darstellt;
Empfangen (802) eines Befehls zum Einstellen einer Region von Interesse (ROI, Region Of Interest) des angezeigten Ultraschallbildes des Herzens an einem Eingabeabschnitt (540); und
Einstellen (803) der ROI des Ultraschallbildes des Herzens basierend auf dem über den Eingabeabschnitt (540) eingegebenen Befehl zum Einstellen der ROI und Vornehmen einer derartigen Steuerung, dass mindestens ein Bild der eingestellten ROI und das Ultraschallbild des Herzens zusammen auf dem Anzeigeabschnitt angezeigt werden,
wobei die Steuerung, dass mindestens ein Bild der eingestellten ROI und das Ultraschallbild des Herzens zusammen angezeigt werden, Folgendes umfasst: Vornehmen einer derartigen Steuerung, dass das Ultraschallbild des Herzens und das mindestens eine ROI-Bild jeweils in geteilten Bereichen angezeigt werden.

10. Verfahren nach Anspruch 9, wobei es sich bei dem Ultraschallbild des Herzens um einen von Langachsenquerschnitten (551a, 553a, 556a) des Herzens handelt, und
wobei es sich bei dem ROI-Bild um mindestens einen von Kurzachsenquerschnitten (551b; 553b, 553c, 553d; 556b) des Herzens handelt.

11. Verfahren nach Anspruch 9, wobei es sich bei dem Ultraschallbild des Herzens um einen von Kurzachsenquerschnitten (552a, 554a) des Herzens handelt, und
wobei es sich bei dem ROI-Bild um mindestens einen von Langachsenquerschnitten (552b; 554b, 554c, 554d) des Herzens handelt.

12. Verfahren nach Anspruch 9, wobei es sich bei dem ROI-Bild um mindestens eines von einem Kurzachsenquerschnittsbild (555b, 555c, 555d) und einem Langachsenquerschnittsbild des Herzens handelt.

## Revendications

1. Appareil échographique (1) comprenant :
un organe de commande (500) conçu pour obtenir une image échographique du coeur sous différents angles et pour en dériver une image stéréoscopique (555a) ;
une partie d'affichage (550) conçue pour afficher l'image échographique du coeur d'un objet, l'image échographique étant l'image stéréoscopique (555a) qui représente le coeur en trois dimensions ;
une partie d'entrée (540) conçue pour recevoir une commande visant à définir une région d'intérêt (ROI, region of interest) de l'image échographique du coeur affichée ;
l'organe de commande (500) étant en outre conçu pour définir la ROI de l'image échographique du coeur en fonction de la commande de définition de la ROI entrée par la partie d'entrée et pour commander au moins une image de la ROI définie et l'image échographique du coeur de façon qu'elles soient affichées ensemble sur la partie d'affichage,
l'organe de commande (500) divisant la partie d'affichage (550) en au moins deux zones et commandant l'image échographique du coeur et l'au moins une image de la ROI de façon qu'elles soient respectivement affichées dans les zones divisées.

2. Appareil échographique (1) selon la revendication 1, dans lequel l'image échographique du coeur est l'une des coupes long axe (551a, 553a, 556a) du coeur, et
dans laquelle l'image de la ROI est au moins l'une des coupes court axe (551b ; 553b, 553c, 553d ; 556b) du coeur.

3. Appareil échographique (1) selon la revendication 1, dans lequel l'image échographique du coeur est l'une des coupes court axe (552a ; 554a) du coeur, et
dans laquelle l'image de la ROI est au moins l'une des coupes long axe (552b ; 554b, 554c, 554d) du coeur.

4. Appareil échographique (1) selon la revendication 1, dans lequel l'image de la ROI est une image en coupe court axe (555b, 555c, 555d) et/ou une image en coupe long axe du coeur.

5. Appareil échographique (1) selon la revendication 1, dans lequel l'organe de commande (500) commande l'image échographique du coeur et une ligne de guidage (U1) liée à la définition de la ROI dans l'image échographique de façon qu'elles soient affichées ensemble.

6. Appareil échographique (1) selon la revendication 1, dans lequel, lorsque la ROI de l'image échographique du coeur est sélectionnée par la partie d'entrée (540), l'organe de commande (500) commande uniquement une image échographique d'une zone correspondant à la ROI sélectionnée à partir de l'image échographique du coeur, de façon qu'elle soit affichée.

7. Appareil échographique (1) selon la revendication 1, dans lequel la partie d'affichage (550) comprend un panneau tactile conçu pour recevoir une commande de sélection de la ROI de l'image échographique du coeur, et
dans lequel, lorsque la ROI de l'image échographique du coeur est sélectionnée par la partie d'affichage (550), l'organe de commande (500) commande l'image échographique du coeur et l'image de la ROI de façon qu'elles soient affichées sur la partie d'affichage (550).

8. Appareil échographique (1) selon la revendication 1, dans lequel l'organe de commande (500) obtient et affiche une liste (551c, 552c) d'une pluralité de telles images de ROI prédéfinies et commande au moins une image de ROI sélectionnée dans la liste de la pluralité d'images ROI affichées de façon qu'elle soit affichée.

9. Procédé de commande d'un appareil échographique (1) à l'aide d'un organe de commande (500), comprenant les opérations consistant à :
obtenir une image échographique du coeur sous différents angles pour en dériver une image stéréoscopique (555a) ;
afficher, sur une partie d'affichage (550), l'image échographique du coeur d'un objet, l'image échographique étant l'image stéréoscopique (555a) qui représente le coeur en trois dimensions ;
recevoir, sur une partie d'entrée (540), une commande visant à définir une région d'intérêt (ROI, region of interest) de l'image échographique du coeur affichée (802) ; et
définir la ROI de l'image échographique du coeur en fonction de la commande de définition de la ROI entrée par une partie d'entrée (540) et commander au moins une image de la ROI définie et l'image échographique du coeur de façon qu'elles soient affichées ensemble (803),
ladite commande d'au moins une image de la ROI définie et de l'image échographique du coeur de façon qu'elles soient affichées ensemble comprenant la commande de l'image échographique du coeur et de l'au moins une image de ROI de façon qu'elles soient respectivement affichées dans des zones divisées.

10. Procédé selon la revendication 9, dans lequel l'image échographique du coeur est l'une des coupes long axe (551a, 553a, 556a) du coeur, et
dans laquelle l'image de la ROI est au moins l'une des coupes court axe (551b ; 553b, 553c, 553d ; 556b) du coeur.

11. Procédé selon la revendication 9, dans lequel l'image échographique du coeur est l'une des coupes court axe (552a, 554a) du coeur, et
dans laquelle l'image de la ROI est au moins l'une des coupes long axe (552b ; 554b, 554c, 554d) du coeur.

12. Procédé selon la revendication 9, dans lequel l'image de la ROI est une image en coupe court axe (555b, 555c, 555d) et/ou une image en coupe long axe du coeur.
